(19) Europäisches Patentamt
European Patent Office
Office européen des brevets

(11) Veröffentlichungsnummer: **0 106 309**
**A2**

(12) # EUROPÄISCHE PATENTANMELDUNG

(21) Anmeldenummer: 83110144.9

(22) Anmeldetag: 11.10.83

(51) Int. Cl.³: A 61 K 35/14
A 61 K 35/20, A 61 K 35/50
A 61 K 35/78, A 61 K 35/72
A 61 K 35/54

(30) Priorität: 12.10.82 DE 3237838
12.10.82 DE 3237837

(43) Veröffentlichungstag der Anmeldung:
25.04.84 Patentblatt 84/17

(84) Benannte Vertragsstaaten:
AT BE CH DE FR GB IT LI LU NL SE

(71) Anmelder: Gauri, Kailash Kumar, Dr. Prof.
D-2359 Lentföhrden(DE)

(71) Anmelder: Möller, Günter, Dr.
D-2205 Dauenhof(DE)

(72) Erfinder: Gauri, Kailash Kumar, Dr. Prof.
D-2359 Lentföhrden(DE)

(72) Erfinder: Möller, Günter, Dr.
D-2205 Dauenhof(DE)

(74) Vertreter: Kinzebach, Werner, Dr.
Patent attorneys Reitstötter, J., Prof.Dr.Dr. Kinzebach, W., Dr. P.O. Box 780
D-8000 München 40(DE)

(54) **Biologisch aktive Extrakte, Verfahren zu deren Herstellung, sie enthaltende pharmazeutische und kosmetische Mittel und ihre Verwendung als Zusätze für Lebens- und Genussmittel.**

(57) Es werden biologisch aktive Extrakte aus Blut, Blutplasma, Milch, Knorpelsubstanz, Plazenta- und Muskelgewebe, Zerealien, Kartoffeln, Fisch, Eiern, Mikroorganismen, wie Hefe, oder Malz beschrieben, die kurzkettige Peptide enthalten.

Diese Extrakte besitzen interessante pharmakologische Eigenschaften und verstärken die Wirkung von bekannten durchblutungsfördernden Wirkstoffen.

EP 0 106 309 A2

Croydon Printing Company Ltd.

Die Erfindung betrifft biologisch aktive Extrakte, die aus dem Gewebe und der Körperflüssigkeit von Säugetieren, aus Zerealien, aus Mikroorganismen oder Eiern gewonnen werden, und pharmazeutische und kosmetische Mittel, die diese Extrakte enthalten. Ein Verfahren zur Herstellung dieser Extrakte und ihre Verwendung als Zusatz zu Lebens- und Genußmitteln ist ebenfalls Gegenstand der Erfindung.

Bekannt ist die Herstellung von biologisch aktiven Extrakten aus dem Blut von Schlachttieren, vgl. z.B. schweizerische Patentschrift 365 483. Bei diesen Extrakten handelt es sich im wesentlichen um die gut wasserlöslichen niedermolekularen Bestandteile des Blutes. Sie haben eine atmungsfördernde Wirkung.

Der Erfindung liegt die Aufgabe zugrunde, biologisch aktive Extrakte mit zellatmungsfördernder Wirkung bereit zu stellen, die in höherer Ausbeute und auf einfache Weise aus leicht zugänglichen Ausgansmaterialien tierischer und pflanzlicher Herkunft oder aus Mikroorganismen gewonnen werden können. Diese Extrakte sollen in kosmetischen und pharmazeutischen Mitteln und als Zusatz zu Lebens- und Genußmitteln die Zellatmung und den Stoffwechsel im Organismus fördern.

Der erfindungsgemäße Extrakt auf der Basis von Blut, Blutplasma, Milch, Knorpelsubstanz, Plazenta- oder Muskelgewebe, Zerealien (z.B. Weizen, Gerste, Roggen oder Reis) Eiern, Hefe, Fisch, Kartoffeln, Mikroorganismen, wie Hefe, oder Malz als Ausgangsmaterial ist dadurch erhältlich, daß man Blut, homogenisierte Knorpelsubstanz, entrahmte Milch, homogenisiertes Plazenta- oder Muskelgewebe, Zerealienkleie, Eier, Fisch, Kartoffeln, Hefe oder Malz mit Wasser auf

einen Feststoffgehalt von etwa 6 bis 14 % einstellt, oder Blutplasma direkt verwendet,

außer bei Verwendung von Malz ein lipolytischesamylytisches-proteolytisches Enzymgemisch zugibt, das aus etwa gleichen Anteilen jeweils eines aus Tier, Pflanze, Pilz und Bakterien gewonnen Enzyms besteht,

unter gleichmäßigem Rühren einige Zeit bei 30-60 °C hält, dann zur Desaktivierung der Enzyme auf etwa 90 °C erhitzt und heiß filtriert,

das Filtrat im Vakuum einengt und das Konzentrat einer Sprühtrocknung unterwirft,

das bei der Sprühtrocknung erhaltene Pulver in einem Alkohol/Wasser-Gemisch suspendiert, die Suspension einige Stunden stehen läßt, dann die überstehende Lösung vom Bodenkörper abfiltriert, das Filtrat konzentriert oder zur Trockne eiengt und gegebenenfalls das so erhaltene Produkt in einem Wasser/Alkohol-Gemisch gegen Wasser dialysiert.

Vorzugsweise stellt man den Trockensubstanzgehalt auf etwa 8 - 10 % ein.

Vorzugsweise verwendet man nach dem ersten Einengungsprozess ein 80/20 Alkohol/Wasser-Gemisch zum Suspendieren und resuspendiert das ausgefallene Material in einem 70/30 Alkohol/Wasser-Gemisch und filtriert das feste Material ab.

Erfindungsgemäß sind auch atmungsaktive Extrakte, ausgehend von Malz erhältlich. Im Falle von Malz liegt das proteolytische Enzymgemisch bereits vor, so daß eine getrennte Zugabe eines derartigen Gemisches nicht erforderlich ist, jedoch möglich ist.

Zur Herstellung des erfindungsgemäßen aktiven Extrakts aus Malz kann man jegliches Malz verwenden, beispielsweise ein handelsübliches Produkt. Zur Herstellung des Extrakts verwendet man beispielsweise gekeimte Gerste, welche getrocknet wurde. Diese wird fein zerkleinert, mit Wasser im Gewichtsverhältnis von ungefähr 1:10 bis 1:15 aufgeschlämmt, unter Rühren langsam im Verlauf von 3 - 4 Stunden auf 90 °C erwärmt. Man setzt Filterhilfe zu, filtriert blank, konzentriert im Vakuum und trocknet im Sprühturm. Die anschließende Verarbeitung besteht im Suspendieren in einem Alkohol/Wasser-Gemsich, Stehenlassen der Suspension, Abfiltrieren der überstehenden Lösung vom Bodenkörper, Konzentrieren des Filtrats bzw. Einengen zur Trockne und gegebenenfalls Dialyse in einem Wasser/Alkohol-Gemisch gegen Wasser.

Bei der erfindungsgemäßen Extrakt handelt es sich um einen peptidhaltigen Extrakt. Die Peptide besitzen ein Molgewicht von etwa 300 - 5000, vorzugsweise 300 - 3000, insbesondere 300 - 1500 und besonders bevorzugt 300 - 1000.

Erfindungsgemäß werden nicht nur Extrakte aus einem Ausgangsmaterial sondern auch Extraktgemische verschiedener Ausgangsmaterialien beansprucht.

Das wie oben beschrieben erhaltene Produkt ist als zell- atmungsfördernder Wirkstoff voll wirksam. Seine Körperverträglichkeit kann durch Dialyse verbessert werden, wenn man das Produkt z.B. für Injektions- oder Infusionszwecke verwenden will. Die so erhaltenen Extrakte enthalten alle niedermolekularen, wasserlöslichen Bestandteile des Blutes, der Milch, der Plazenta und der Zerealienkleie. Generell haben sie eine zellatmungssteigernde Wirkung, die sich im Warburg-Test* nachweisen läßt. Sie können daher als Durchblutungsstörungen kompensierende, die Wundheilung beschleunigende Mittel und zur Antagonisierung von oxygenasehemmenden Stoffen, wie Acetylsalicylsäure, Flufenamic acid, Nefenamic acid oder Indomethacin in der Therapie verwendet werden. Es wurde auch gefunden, daß sie die toxischen Wirkungen von Glycolyse-Hemmern, wie Phosphonaten, z.B. Phosphonoessigsäure oder Arabinofuranosyladenin bzw. dessen 5'-Phosphat, antagonisieren und antiviral und analgetisch wirken. Außerdem können sie vorteilhaft in der Kosmetik verwendet werden, beispielsweise zur Herstellung von Hautcremes und Lotionen.

* Der Warburg-Test ist beschrieben in Leuthardt, "Lehrbuch der physiologischen Chemie," 1959, Seiten 220 und 221, Walter de Gruyter & Co., Berlin.

Es wurde überraschenderweise gefunden, daß das erfindungsgemäße Produkt eine Reihe von toxischen Effekten stark antagonisiert. Im Mäuseversuch kann man dies z.B. am Pentoxifyllin zeigen. Eine Pentoxifyllin-Dosis von etwa 300 mg/kg, i.p., ist normalerweise tödlich für eine Maus. Verabreicht man jedoch etwa 30 Minuten vorher das erfindunsgemäße Produkt in einer Dosis von etwa 400 mg/kg intraperitoneal oder intravenös, so erreicht man damit einen Schutzeffekt, der sich in einer entsprechenden Erniedrigung der Todesrate ausdrückt. Die auf diese Weise ermittelten Aktivitäten der erfindungsgemäßen Extrakte sind in der nachfolgenden Tabelle zusammengestellt und einem bekannten atmungsfördernden Wirkstoff gegenüber gestellt:

| Schützende Substanz (400 mg/kg) | Sterblichkeit durch 300 mg/kg Pentoxifyllin % | Schutzeffekt % |
|---|---|---|
| Kontrolle: 0,9 % NaCl-Lösung | 100 | 0 |
| Blutextrakt gemäß Beispiel 1, i.p. | 0 | 100 |
| Milchextrakt gemäß Beispiel 2, i.p. | 20 | 80 |
| Weizenkleieextrakt gemäß Beispiel 5 | 30 | 70 |
| Blutextrakt gemäß der CH-PS 365 483, i.p. | 80 | 20 |

Der bekannte Vergleichsextrakt ist ein Infusionspräparat auf der Basis von deproteinisiertem Kälberblut, das bei schweren zerebralen Durchblutungs- und Stoffwechselstörungen sowie peripheren arteriellen und venösen Durchblutungsstörungen und Verbrennungen verabreicht wird.

Die oben erwähnten Extrakte wurden als 4 %ige Lösungen intraperitoneal verabreicht, etwa 30 Minuten später wurde die toxische Dosis Pentoxifyllin in 3 %iger Lösung ebenfalls intraperitoneal verabreicht. Als Kontrolle diente eine 0,9 %ige NaCl-Lösung. Das Volumen der verabreichten Lösungen wurde konstant gehalten, es betrug immer 0,1 ml/10 g Maus. Die erfindungsgemäßen Extrakte besitzen volle Wirksamkeit, auch ohne zusätzliche Reinigung durch Dialyse.

Weitere Untersuchungen in dieser Richtung bestätigten, daß durch die Kombination eines atmungssteigernden natürlichen Wirkstoffes der oben bezeichneten Art mit einem durchblutungsfördernden Wirkstoff, der therapeutische Index des durchblutungsfördernden Wirkstoffes erheblich gesteigert werden kann.

Als durchblutungsfördernden Wirkstoff verwendet man z. B.:

i) das 2,5-Dihydroxibenzolsulfonsäure-calciumsalz, auch Calciumdobesilat genannt;

ii) ein 3,7-Dihydro-3,7-di-$C_{1-4}$-alkyl-1-(5-substituiertes)-1H-purin-2,6-dion der Formel I:

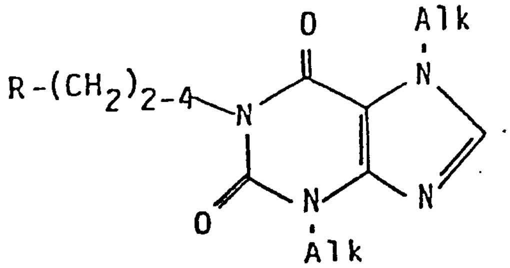

(I)

worin Alk für gleiche oder verschiedene gerade oder verzweigte Niedrigalkylreste mit 1 bis 4 C-Atomen und

R für einen $-\underset{\underset{O}{\|}}{C}-CH_3$ oder $-C_2H_5$ Rest steht;

iii) ein Pyrazolo [3,4-d] pyrimidin der allgemeinen Formel II:

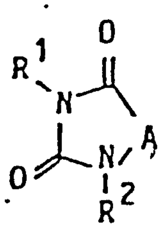

(II),

worin

$R^1$ und $R^2$, die gleich oder verschieden sind, für ein H-Atom oder die Reste $C_1$-$C_8$-Alkyl, $C_2$-$C_8$-Alkenyl, $C_4$-$C_8$-Alkylcarbonylalkyl, $R^1$ und $R^2$ jedoch nicht gleichzeitig ein H-Atom sein können und

A für eine Gruppe

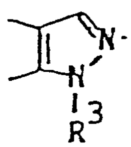 oder 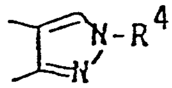 ,

stehen, wobei
$R^3$ ein H-Atom oder $C_1$-$C_4$-Alkyl und
$R^4$ ein H-Atom, $C_1$-$C_6$-Alkyl oder $C_2$-$C_6$-Alkenyl bedeuten. Vorzugsweise stehen
$R^1$ und $R^3$ für Methyl oder Äthyl und
$R^2$ für $-(CH_2)_4-CO-CH_3$.

iv) ein Pyridon der Formel III:

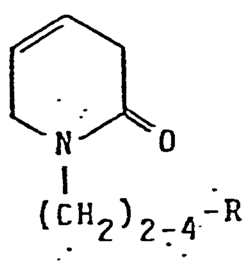 (III)

worin R einen $-\underset{\underset{O}{\|}}{C}-CH_3$ , $-C_2H_5$ oder $-C_2H_4OH$ Rest bedeutet;

v) oder Nicergolin, Vincamin oder Dipyridamol.

Bevorzugte Verbindungen der Formel I sind Pentoxifylline und Pentifylline.

Die Wirkstoffe der Absätze i), ii) und v) sind bekannt, vgl. z. B. "The Merck Index", 9. Auflage, Nr. 3406, 6927, 6931, 9638, 6310 und 3366.

Die Pyrazolopyrimidine der allgemeinen Formel II werden hergestellt, indem man ein 4-Hydrazinouracil der allgemeinen Formel IIa:

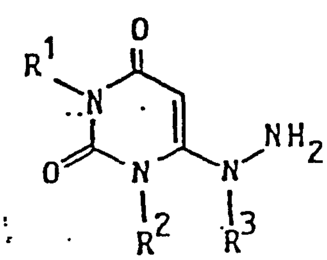

(IIa)

worin $R^1$, $R^2$ und $R^3$ die oben angegebenen Bedeutungen besitzen, mit Phosphoroxychlorid und Dimethylformamid in an sich bekannter Weise, beispielsweise nach dem

in der DE-AS 1 186 466 angegebenen Verfahren, umsetzt. Vorteilhafterweise trägt man dabei das 4-Hydrazinouracil in eine gekühlte Mischung aus Phosphoroxychlorid und Dimethylformamid ein. Wenn das 4-Hydrazinouracil vollständig eingetragen worden ist, läßt man die Reaktionsmischung sich auf Raumtemperatur erwärmen. Beim Eintragen von Eis fällt das gewünschte Produkt aus.

Wenn einer der Reste $R^1$ oder $R^2$ für einen Alkylcarbonylalkylrest steht, kann man vorteilhafterweise so vorgehen, daß man ein entsprechendes 4,5,6,7-Tetrahydro-4,6-dioxo-pyrazolo [3,4-d] pyrimidin mit einem ω,ω'-Dibromalkan alkyliert, das erhaltene ω-Bromalkylderivat mit Acetessigester umsetzt und das erhaltene Produkt einer Ketonspaltung unterwirft. Beispielsweise erhält man durch Umsetzung von 7-Ethyl-4,5,6,7-tetrahydro-1-methyl-4,6-dioxo-1H-pyrazolo[3,4-d]pyrimidin mit 1,3-Dibrompropan das 5-(3-Brompropyl)-7-ethyl-4,5,6,7-tetrahydro-1-methyl-4,6-dioxo-1H-pyrazolo[3,4-d]pyrimidin, aus dem man durch Umsetzung mit Acetessigester und anschließende Ketonspaltung das 7-Ethyl-4,5,6,7-tetrahydro-1-methyl-4,6-dioxo-5-(5-oxo-n-hexyl)-1H-pyrazolo[3,4-d] pyrimidin erhält. Der Alkylcarbonylalkylrest kann andererseits auch durch Umsetzung eines 4,5,6,7-Tetrahydro-4,6-dioxo-pyrazolo[3,4-d]pyrimidins, vorzugsweise als Natriumsalz, mit einem Alkylcarbonylalkylhalogenid, vorzugsweise einem Chlorid, eingeführt werden. Zum Beispiel kann der 5-Oxo-n-hexylrest durch 1-Chlorhexanon-5 eingeführt werden.

Die Pyridone der Formel III sind aus den deutschen Patentanmeldungen P 22 28 289 und P 22 25 229 bekannt.

Beispiele für bevorzugte Pyrazolopyrimidine der Formel II sind:
7-n-Hexyl-4,5,6,7-tetrahydro-1,5-dimethyl-4,6-dioxo-1H-pyrazolo[3,4-d]-pyrimidin (Schmp. 111°C);
5-n-Hexyl-4,5,6,7-tetrahydro-1,7-dimethyl-4,6-dioxo-1H-pyrazolo[3,4-d]-pyrimidin;
7-i-Butyl-4,5,6,7-tetrahydro-1,5-dimethyl-4,6-dioxo-1H-pyrazolo[3,4-d]-pyrimidin;
2-Ethyl-5-n-hexyl-4,5,6,7-tetrahydro-7-methyl-4,6-dioxo-2H-pyrazolo-[3,4-d]pyrimidin (Schmp. 110°C);
2-n-Butyl-7-n-hexyl-4,5,6,7-tetrahydro-5-methyl-4,6-dioxo-2H-pyrazolo-[3,4-d]pyrimidin (Schmp. 67°C);
4,5,6,7-Tetrahydro-5-methyl-4,6-dioxo-7-(5-oxo-n-hexyl)-2H-pyrazolo-[3,4-d]pyrimidin;
4,5,6,7-Tetrahydro-1-methyl-4,6-dioxo-5-(5-oxo-n-hexyl)-3-n-propyl-1H-pyrazolo[3,4-d]pyrimidin;
5-n-Hexyl-4,5,6,7-tetrahydro-2,7-dimethyl-4,6-dioxo-2H-pyrazolo[3,4-d]-pyrimidin (Schmp. 73-75°C);
2-n-Butyl-5-n-hexyl-4,5,6,7-tetrahydro-7-methyl-4,6-dioxo-2H-pyrazolo-[3,4-d]pyrimidin (Schmp. 88-90°C);
2,5-Di-n-hexyl-4,5,6,7-tetrahydro-7-methyl-4,6-dioxo-2H-pyrazolo[3,4-d]-pyrimidin (Schmp. 80-81°C);
7-n-Hexyl-4,5,6,7-tetrahydro-5-methyl-4,6-dioxo-2H-pyrazolo[3,4-d]-pyrimidin (Schmp. 159-161°C)
7-n-Hexyl-4,5,6,7-tetrahydro-2,5-dimethyl-4,6-dioxo-2H-pyrazolo[3,4-d]-pyrimidin (Schmp. 109-110°C);
2,7-Di-n-hexyl-4,5,6,7-tetrahydro-5-methyl-4,6-dioxo-2H-pyrazolo[3,4-d]-pyrimidin (Schmp. 96-97°C);
4,5,6,7-Tetrahydro-2-methyl-4,6-dioxo-7-(5-oxo-n-hexyl)-2H-pyrazolo-[3,4-d]pyrimidin;

7-Allyl-2-n-hexyl-4,5,6,7-tetrahydro-1,5-dimethyl-4,6-dioxo-2H-pyrazolo-[3,4-d]pyrimidin;
4,5,6,7-Tetrahydro-3,5-dimethyl-7-(2-methylbutyl)-4,6-dioxo-1H-pyrazolo[3,4-d]pyrimidin;
4,5,6,7-Tetrahydro-1,5-dimethyl-7-(2-methylbutyl)-4,6-dioxo-1H-pyrazolo[3,4-d]pyrimidin;
7-n-Hexyl-4,5,6,7-tetrahydro-5-methyl-4,6-dioxo-2-n-propyl2H-pyrazolo-[3,4-d]pyrimidin;
4,5,6,7-Tetrahydro-1,5-dimethyl-4,6-dioxo-7-(5-oxo-n-hexyl)-1H-pyrazolo[3,4-d]pyrimidin;
2-Ethyl-7-n-hexyl-4,5,6,7-tetrahydro-5-methyl-4,6-dioxo-2H-pyrazolo[3,4-d]pyrimidin (Schmp. 96-97°C);
5-n-Hexyl-4,5,6,7-tetrahydro-7-methyl-4,6-dioxo-1H-pyrazolo[3,4-d]pyrimidin (Schmp. 138°C);
3-Ethyl-5-n-hexyl-4,5,6,7-tetrahydro-1,7-dimethyl-4,6-dioxo-1H-pyrazolo[3,4-d]pyrimidin;
5-n-Hexyl-4,5,6,7-tetrahydro-3-(3,4,5-trimethoxyphenyl)-1,7-dimethyl-4,6-dioxo-1H-pyrazolo[3,4-d]pyrimidin;
7-i-Butyl-4,5,6,7-tetrahydro-5-methyl-4,6-dioxo-1H-pyrazolo[3,4-d]pyrimidin (Schmp. 204°C);
4,5,6,7-Tetrahydro-3-(2-hydroxyethyl)-4,6-dioxo-5-(3-oxobutyl)-2H-pyrazolo[3,4-d]pyrimidin.

Die erfindungsgemäßen atmungssteigernden Extrakte können daher vorteilhaft in Arzneimitteln verwendet werden, die einen oder mehrere der oben genannten durchblutungsfördernden Wirkstoffe enthalten, gegebenenfalls in einem üblichen festen oder flüssigen Arzneimittelträger.

Die erfindungsgemäßen Extrakte können auch mit Calcium-Antagonisten kombiniert werden. Es wurde nun überraschend gefunden, daß die pharmakologischen Wirkungen von Calcium-Antagonisten durch den Einsatz der erfindungsgemäßen Extrakte in synergistischer Weise gesteigert werden können.

Dies kann anhand der Verbesserung des Schutzeffektes gegenüber einer Schädigung retinaler Funktionen durch Allylalkohol gezeigt werden.

Die netzhautschädigende Wirkung von Allylalkohol läßt sich mit Hilfe des Elektroretinograms der Augennetzhaut nachweisen. Die elektroretinographische Erfassung dieser Schädigung entspricht der von GAURI et al. in DOCUMENTA OPHTHALMOLOGICA; The Hague 1977, Seite 335 bis 340 und in "Der Augenspiegel", Heft 9, 1979, Seiten 1 bis 16, beschriebenen Methodik.

Um den schädigenden Einfluß von Allylalkohol zu untersuchen, wurde dunkel adaptierten weiblichen NMRI-Mäusen 25 Minuten vor einer Urethannarkose 100 mg/kg i.p. Allylalkohol injiziert. Die Tiere wurden dann innerhalb von 5 Minuten an ein Elektroretinogramm-(ERG)-Gerät angeschlossen. Dazu wurde eine Silberchlorid-Watte-Elektrode auf die Hornhautoberfläche der Versuchstiere aufgelegt und eine zweite Silbernadel-Elektrode in den Lidwinkel des Auges des Versuchstiere retrobulbär eingestochen. Die Registrierung der ERG-b-Wellenpotentiale erfolgte bei voller Lichtstärke. Auf diese Weise wurden die maximalen b-Wellenpotentiale ermittelt.

Zur Prüfung des Schutzeffektes erhielten die dunkeladaptierten Tiere zuerst die Wirkstofflösungen i.p. injiziert. 30 Minuten später erfolgte die Verabreichung von Allylalkohol 100 mg/kg i.p., weitere 25 Minuten später wurden die Tiere mit Urethan narkotisiert und innerhalb von 5 Minuten, wie üblich, an das ERG-Gerät angeschlossen.

Die erhaltenen Ergebnisse sind in der folgenden Tabelle zusammengefaßt.

Verbesserung des Schutzeffektes durch die Kombination von Ca-Antagonisten mit den erfindungsgemäßen Extrakten gegenüber der Allylalkoholschädigung gezeigt am Elektroretinogramm der Maus

| Vorbehandlung und Wirkstoff | | maximale b-Wellen Potentiale |
|---|---|---|
| Kontrolle (Urethannarkose) | | 1 326 |
| Schädigung mit Allylalkohol | | 365 |
| Nimodepin (A) 10 mg/kg p.o. | | 707 |
| Rinderblutextrakt(B) 10 mg/kg i.p. | Erfindung | 690 |
| Kombination aus (A) und (B) | " | 1 503,5 |
| Diltiazem (C) (10 mg/kg i.p.) | | 585 |
| Kombination aus (B) und (C) | " | 1 177 |

Diese Ergebnisse zeigen, daß diejenigen Tiere, denen ein erfindungsgemäßer Extrakt zusammen mit einem Ca-Antagonisten verabreicht worden ist, gegen die durch Allylalkohol hervorgerufene Schädigung fast völlig geschützt sind, während die nur mit einem Wirkstoff behandelten Tiere nur sehr wenig geschützt sind.

Die erfindungsgemäßen Extrakte haben eine überraschend vorteilhafte antivirale Wirkung, auch ohne andere therapeutische Zusätze. Typische Herpes Erkrankungen (Herpes zoster, genitalis, labialis und corneae) können sehr erfolgreich damit behandelt werden. Überraschenderweise bleibt diese Wirkung auch bei oraler Applikation erhalten.

Die erfindungsgemäßen Arzneimittel können parenteral, vorzugsweise in Form von wäßrigen Lösungen, mit oder ohne Lösungsvermittler oder oral in festen oder flüssigen Formulierungen, z. B. als Tabletten, Dragees oder wäßrige Lösungen appliziert werden. Gewünschtenfalls können sie auch als lokal anwendbare Präparate, z. B. als Salbe, Gel, Puder oder Lotion zubereitet werden. Sie können auch als Inhalationspräparate formuliert sein, z. B. als Spray.

Im Hinblick auf die Tatsache, daß kreislauffördernde Mittel in der Regel über längere Zeit (meist mehrere Jahre) angewendet werden, ist die Herabsetzung der Toxizität dieser Verbindungen durch die erfindungsgemäße Kombination eine wesentliche Bereicherung der Humantherapie.

Nach einer anderen bevorzugten Ausführungsform betrifft die Erfindung die Verwendung des zuvor gekennzeichneten atmungsaktiven Extraktes als Zusatz zu Bier und anderen alkoholischen Getränken. Besonders bevorzugt ist die Verwendung einer Menge von 0,2 bis 10 Gew.-% an atmungsaktivem Extrakt, bezogen auf das Gesamtgewicht des jeweiligen Lebens- und Genußmittels.
Besonders bevorzugt sind Zusätze in einer Menge von 0,5 bis 7 Gew.-%, insbesondere 1 bis 4 Gew.-%.

In Lebens- und Genußmitteln finden sich häufig Bestandteile, welche die normale Zellatmung und den Stoffwechsel nachteilig beeinflussen, beispielsweise Oxygenase-Hemmer und Glycolyse-Hemmer. Häufig werden Nahrungsmittel angeboten, welche unerwünschte Spuren an Schwermetallsalzen und anderen toxischen Stoffen enthalten. Diese Stoffe müssen durch aktiven Sauerstoff aus dem Organismus eleminiert werden.

So erfolgt beispielsweise beim kurzzeitigen und insbesondere längeren Genuß von Alkoholen die Bildung von Aldehyden, insbesondere von Acetaldehyd, welche für den Organismus schädlich sind.
Zur Entgiftung von Aldehyden stehen dem Organismus Oxydasen zur Verfügung, welche unter Verwendung von Sauerstoff die Aldehyde abbauen. Durch Anreicherung von atmungsaktiven Stoffen in Lebens- und Genußmitteln läßt sich die für den Organismus wichtige Detoxifikation erzielen.

Die erfindungsgemäße Verwendung des atmungsaktiven Extrakts führt zu einer derartigen Detoxifizierung des Organismus. Die toxischen glycolyse- bzw. oxydasehemmenden Effekte von toxischen Stoffen in den Lebens- und Genußmitteln werden durch die erfindungsgemäße Verwendung verhindert bzw. kompensiert.

Im Bier liegen bereits Stoffe vor, welche in gewißem Maße die vorstehenden toxischen Effekte mildern. Die erfindungsgemäße Verwendung der atmungsaktiven Extrakte als Zusatz zum Bier führt zu einer wesentlichen Steigerung der detoxifizierenden Wirkung des Biers.

Es wird angenommen, daß die die Zellatmung und den Stoffwechsel fördernde Wirkung bei der erfindungsgemäßen Verwendung darauf beruht, daß die energieliefernden Prozesse im Organismus unterstützt werden. Die erfindungsgemäße Verwendung führt zu unerwartet vorteilhaften ernährungsphysiologischen Effekten der vorstehend erläuterten Art.

Die nachfolgenden Beispiele dienen zur weiteren Erläuterung der Erfindung.

Beispiel 1

Dickblut vom Schlachthof wird vom Plasma befreit und mit Wasser auf einen Feststoffgehalt von etwa 10 Gew.-% verdünnt. Zu 10 l dieses Blutes gibt man etwa 10 g eines amylytischen-lipolytischen-proteolytischen Enzymgemisches, z.B. ein Gemisch aus gleichen Anteilen Pankreatin, Papain und zwei aus Hefekulturen gewonnenen Enzymen. Unter gleichmäßigem, langsamem Rühren wird auf 35-38°C erwärmt. Unter ständigem, gleichmäßigem Rühren hält man dieses Temperaturniveau etwa 1 Stunde lang, erwärmt dann weiter auf etwa 53-57°C, hält auf diesem Temperaturniveau etwa 2 Stunden lang, gibt schließlich ein Filterhilfsmittel zu, erhitzt auf etwa 90° C und filtriert heiß durch eine Filterpresse. Das erhaltene klare Filtrat wird im Vakuum auf ca. 30 % Festsubstanz eingeengt und anschließend sprühgetrocknet. Das erhaltene Pulver suspendiert man in einem 70/30 Alkohol/Wasser-Gemisch, dabei stellt man einen Feststoffgehalt von etwa 10 Gew.-% ein. Man läßt die Suspension etwa 8-12 Stunden stehen, dabei setzen sich die ungelöstenBestandteile ab. Man filtriert die klare überstehende Lösung, dampft den Alkohol ab und unterwirft den Rückstand einer Sprühtrocknung. Das so erhaltene Produkt ist farblos und für die erfindungsgemäßen Zwecke bereits voll verwendbar. Zur Verbesserung der Körperverträglichkeit kann man dieses Produkt durch Dialyse weiter reinigen. Vorteilhaft dialysiert man ein 70/30 Alkohol-Wasser-Gemisch gegen Wasser. Das durch Dialyse gereinigte Produkt kann ohne weiteres für Injektions- und Infusionszwecke verwendet werden.

Beispiel 2

10 l entrahmter Frischmilch werden mit je 5 g Pankreatin und Papain und zwei aus Pilzen und Bakterien gewonnenen Enzymen versetzt. Unter ständigem, langsamem Rühren erwärmt man auf etwa 36-38°C und bleibt auf diesem Temperaturniveau etwa 1 Stunde lang. Allmählich entfärbt sich die Milch und man erhält eine blanke Lösung. Nun hält man die Temperatur auf etwa 54-56°C, bleibt unter ständigem Rühren auf diesem Temperaturniveau, gibt anschließend ein Filterhilfsmittel zu, erhitzt auf etwa 90°C und filtriert heiß durch eine Filterpresse. Das klare Filtrat wird z. B. in einem Wiegand-Fallstromverdampfer konzentriert und in einem Sprühverdampfer getrocknet. Das so erhaltene Produkt suspendiert man in einer wäßrigen alkoholischen Lösung (etwa 80 Gew.-% Äthanol / 20 Gew.-% Wasser), läßt diese Suspension dann etwa 12 Stunden lang stehen, filtriert, entfernt den Alkohol und unterwirft den Rückstand nochmals der Sprühtrocknung. Man erhält ein farbloses, für die erfindungsgemäßen Zwecke geeignetes Produkt. Zur weiteren Reinigung kann man es einer Dialyse unterwerfen.

Beispiel 3

1,6 kg Magermilchpulver werden in 20 l entmineralisiertem Wasser kalt gelöst. Nach mehrstündigem Rühren erhält man eine homogene Lösung. Diese wird analog den Angaben des Beispiels 2 weiter verarbeitet. Man erhält ein farbloses voll aktives Produkt.

Beispiel 4

Plazentagewebe von Schlachttieren wird in einem Homogenisator zerkleinert, mit Wasser wird verdünnt und auf einen Feststoffgehalt von etwa 10 Gew.-% eingestellt. Die Weiterverarbeitung erfolgt nach den Angaben des Beispieles 1. Man erhält ein farbloses, voll wirksames Produkt.

Beispiel 5

10 kg Weizenkleie werden fein vermahlen und in 100 Liter kaltem destilliertem Wasser suspendiert. In diesen Zustand gibt man das Enzymgemisch des Beispieles 1 zu und erwärmt unter ständigem Rühren auf maximal 35°C. Man hält eine Stunde die Temperatur konstant und erhöht auf 55-60°C. Wiederum wird 2 Stunden bei dieser Temperatur gerührt und anschließend auf 90° erhitzt, mit Filtererde versetzt und durch eine Filterpresse geklärt. Die Lösung wird im Vakuum eingeengt, mit Alkohol/Wassergemisch (70/30) versetzt und vom ausfallenden Rückstand getrennt. Nach Abdampfen des Alkohols wird die Lösung sprühgetrocknet.

Das erfindungsgemäße Produkt kann in Bezug auf seine biologische Aktivität standardisiert werden.

Beispiel 6

(Extrakt aus Malz)
Man zermahlt 10 kg gekeimte und getrocknete Gerste und suspendiert in 100 l kaltem destilliertem Wasser. Dann erwärmt man unter ständigem Rühren im Verlauf von 3 1/2 Stunden auf eine Endtemperatur von etwa 90° C. Man versetzt mit Filterhilfe und klärt durch eine Filterpresse. Die erhaltene Lösung wird im Vakuum eingeengt, mit Alkohol/Wasser-Gemisch (70/30)

versetzt und vom ausfallenden Rückstand abgetrennt. Nach dem Abdampfen des Alkohols wird die Lösung sprühgetrocknet.

Beispiel 7

10 kg Hefe suspendiert man in 100 l destilliertem Wasser, zerstört die Zellen durch Ultraschall, gibt unter ständigem Rühren das in Beispiel 1 beschriebene Enzymgemisch zu und erhöht die Temperatur langsam bis auf maximal 37 °C und hält bei dieser Temperatur etwa 1 Stunde lang. Danach erhöht man die Temperatur auf 55 bis 60 °C und hält 2 Stunden wie üblich auf dieser Temperatur. Man gibt Filterhilfe zu, erhitzt die Mischung dann auf 90 °C und behandelt das klare Filtrat wie in den anderen Beispielen beschrieben, wobei man das aktive Produkt erhält.

Beispiel 8

Man homogenisiert 10 kg angebrütete Eier zusammen mit den Schalen, verdünnt mit Wasser auf 100 kg, behandelt mit Enzymen bei erhöhter Temperatur, wobei man ein klares Filtrat erhält. Man reinigt durch Ausfällen mit Äthanol-Wasser (70:30).

Beispiel 9

Man behandelt 10 kg frische Eier wie in Beispiel 8 beschrieben, wobei man das aktive Produkt erhält.

Beispiel 10

5 kg Kartoffelreste (Schalen), erhalten als Abfallprodukt von der Kartoffelindustrie, schlämmt man mit 50 l Wasser auf und homogenisiert mechanisch. Die Mischung kocht man 30 Minuten, kühlt dann auf 30 °C, gibt das Enzymgemisch zu und erhäht die Temperatur auf 37 °C. Diese Temperatur hält man eine Stunde und erhöht dann die Temperatur für 2 Stunden auf 60 °C. Dann gibt man Filterhilfe zu und dampft die Mischung ein. Man verfährt weiter wie oben beschrieben, wobei man einen aktiven Extrakt erhält.

Anwendungsbeispiele

1) Gleiche Mengen an Calciumdobesilat und Pentoxyfylline wurden in einem etwa 0,02 bis 0,6 %-igen wäßrigen Blutextrakt gemäß Beispiel 1 gelöst. Die Einzeldosis enthielt etwa 60 bis 100 mg Dobesilat, 25 bis 100 mg Pentoxyfylline und 1 bis 100 mg Blutextrakt.

Ein Präparat analoger Zusammensetzung kann durch Vermischen der entsprechenden Mengenanteile Festsubstanz und einem üblichen, pharmazeutisch verträglichen Träger hergestellt werden. Analoge Präparate können aus dem oben erwähnten Blutextrakt und den anderen in der vorliegenden Anmeldung bezeichneten durchblutungsfördernden

Wirkstoffen, wie Vincamin, Nicergolin, einem der hier bezeichneten Pyrazolopyrimidine oder Pyridone hergestellt werden. Für die Herstellung der erfindungsgemäßen Präparate verwendet man dabei etwa 1/4 bis 1/10 der üblichen therapeutischen Dosis der jeweiligen Wirkstoffe in Kombination mit dem hier bezeichneten atmungssteigernden natürlichen Wirkstoff.

2) Injektionslösung:

| | |
|---|---|
| Pentoxifyllin | 40 mg |
| Blutextrakt | 20 mg |
| Isotonische NaCl-lösung ad | 5 ml |

3) Infusionslösung:

| | |
|---|---|
| Vincamin | 800 mg |
| Blutextrakt | 3000 mg |
| 10 %ige wäßrige Glucoselösung ad | 1 l |

4) 1000 Tabletten:

| | |
|---|---|
| Pentifyllin | 100 g |
| Blutextrakt | 20 g |
| Trägermasse für Tablette | 80 g |

5) Anti-Herpes Mittel:

| | |
|---|---|
| Blutextrakt | 5 g |
| Isopropanol/$H_2O$ 60/40 ad | 100 ml |

--

## Patentansprüche

1. Extrakt auf der Basis von Blut, Blutplasma, Milch, Knorpelsubstanz, Plazenta- und Muskelgewebe, Zerealien, Kartoffeln, Fisch, Eiern, Mikroorganismen, wie Hefe, oder Malz als Ausgangsmaterial, dadurch erhältlich, daß man Blut, homogenisierte Knorpelsubstanz, entrahmte Milch, homogenisiertes Plazenta- oder Muskelgewebe, Zerealienkleie, Eier, Fisch, Kartoffeln, Hefe oder Malz mit Wasser auf einen Feststoffgehalt von etwa 6 bis 14 % einstellt, oder Blutplasma direkt verwendet,

   außer bei Verwendung von Malz ein lipolytischesamylytisches-proteolytisches Enzymgemisch zugibt, das aus etwa gleichen Anteilen jeweils eines aus Tier, Pflanze, Pilz und Bakterien gewonnen Enzyms besteht,

   unter gleichmäßigem Rühren einige Zeit bei 30-60$^{\circ}$ C hält, dann zur Desaktivierung der Enzyme auf etwa 90 $^{\circ}$C erhitzt und heiß filtriert,

   das Filtrat im Vakuum einengt und das Konzentrat einer Sprühtrocknung unterwirft,

   das bei der Sprühtrocknung erhaltene Pulver in einem Alkohol/Wasser-Gemisch suspendiert, die Suspension einige Stunden stehen läßt, dann die überstehende Lösung vom Bodenkörper abfiltriert, das Filtrat konzentriert oder zur Trockne einengt und gegebenenfalls das so erhaltene Produkt in einem

Wasser/Alkohol-Gemisch gegen Wasser dialysiert;

und Extraktgemische aus Extrakten der verschiedenen obigen Ausgangsmaterialien.

2. Extrakt nach Anspruch 1, dadurch erhältlich, daß man als Enzymgemisch ein Gemisch aus etwa gleichen Anteilen Pankreatin, Papain, Pilzprotease und Bakterienprotease verwendet.

3. Extrakt nach Anspruch 1 oder 2, dadurch erhältlich, daß man ein etwa 70/30 Alkohol/Wasser-Gemisch zum Suspendieren des nach dem ersten Einengungsprozesses erhaltenen Produktes verwendet und dabei auf einen Feststoffgehalt von etwa 10 % einstellt.

4. Extrakt nach Anspruch 3, dadurch erhältlich, daß man bei der Herstellung des Extraktes ein 80/20 Alkohol/Wasser-Gemisch verwendet und das so erhaltene Präzipitat in einem 70/30 Alkohol/Wasser-Gemisch resuspendiert und die klare Alkohollösung zur Trockene einengt.

5. Pharmazeutische und kosmetische Mittel bestehend aus mindestens einem oder enthaltend mindestens einen Extrakt nach einem der Ansprüche 1 bis 4.

6. Pharmazeutisches Mittel nach Anspruch 5, enthaltend mindestens einen Extrakt nach einem der Ansprüche 1 bis 4 und mindestens einen vasoaktiven Wirkstoff, gegebenenfalls in einem üblichen pharmazeutisch verträglichen Verdünnungsmittel.

7. Pharmazeutisches Mittel enthaltend mindestens einen Extrakt nach einem der Ansprüche 1 bis 4 in Kombination mit mindestens einem der folgenden Wirkstoffe:

A) einem vasoaktiven Wirkstoff, nämlich:

i) 2,5-Dihydroxibenzolsulfonsäure-calciumsalz, auch Calciumdobesilat genannt;

ii) ein 3,7-Dihydro-3,7-di-$C_{1-4}$-alkyl-1-(5-substituiertes)-1H-purin-2,6-dion der Formel I:

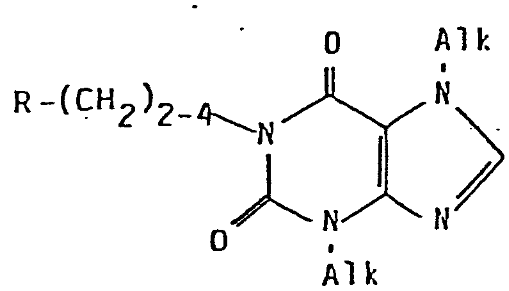

(I)

worin Alk für gleiche oder verschiedene, gerade oder verzweigte Niedrigalkylreste mit 1 bis 4 C-Atomen und

R für einen $-\underset{\overset{\|}{O}}{C}-CH_3$ oder $-C_2H_5$ Rest steht;

iii) ein Pyrazolo[3,4-d]pyrimidin der allgemeinen Formel II:

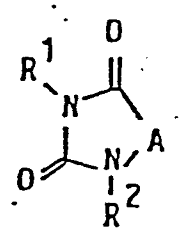

(II),

worin

$R^1$ und $R^2$, die gleich oder verschieden sind, für ein H-Atom oder die Reste $C_1$-$C_8$-Alkyl, $C_2$-$C_8$-Alkenyl, $C_4$-$C_8$-Alkylcarbonylalkyl, wobei $R^1$ und $R^2$ jedoch nicht gleichzeitig ein H-Atom sein können, und

A für eine Gruppe

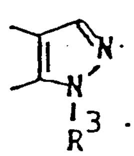

oder

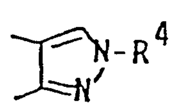

,

stehen, wobei

$R^3$ ein H-Atom oder $C_1$-$C_4$ Alkyl und

$R^4$ ein H-Atom, $C_1$-$C_6$-Alkyl oder $C_2$-$C_6$-Alkenyl bedeuten,

vorzugsweise stehen $R^1$ und $R^3$ für Methyl oder Äthyl und $R^2$ für $-(CH_2)_4-CO-CH_3$;

iv) ein Pyridon der Formel III:

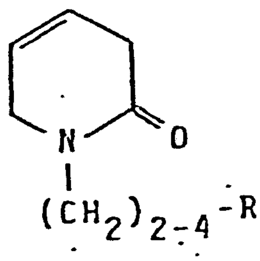

(III)

worin R einen $-\underset{\underset{O}{\|}}{C}-CH_3$ , $-C_2H_5$ oder $-C_2H_4OH$ Rest bedeutet;

v) oder Nicergolin, Vincamin oder Dipyridamol,

B) einem Oxygenase-Hemmer, nämlich Acetylsalicylsäure, Flufenamicacid, Mefenamicacid und Indomethacin,

C) einem Glycolyse-Hemmer, nämlich Phosphonoessigsäure, Arabinofuranosyladenin bzw. dessen 5'-Phosphat,

D) einem Calcium-Antagonisten.

8. Verwendung des Extraktes nach einem der Ansprüche 1 bis 4 als Zusatz zu Lebens- und Genußmitteln.

9. Verwendung nach Anspruch 8 als Zusatz zu Bier und anderen alkoholischen Getränken, oder als Zusatz zu nicht-alkoholischen Getränken, Brot, Kartoffelprodukten, Fleisch, Fisch, Wurstwaren, Obst und Gemüse, sowie deren Weiterverarbeitungsprodukten, Milch- und Käseprodukten, Süßwaren, Speiseeis.

10. Verwendung nach Anspruch 8 in einer Menge von 0,2 bis 10 Gew.-%, vorzugsweise in einer Menge von 0,5 bis 7 Gew.-%, insbesondere bevorzugt in einer Menge von 1 bis 4 Gew.-%.

11. Verfahren zur Herstellung eines Extraktes nach Anspruch 1, dadurch gekennzeichnet, daß man Blut, homogenisierte Knorpelsubstanz, entrahmte Milch, homogenisiertes Plazenta- oder Muskelgewebe, Zerealienkleie, Eier, Hefe, Fisch, Kartoffeln oder Malz mit Wasser auf einen Feststoffgehalt von etwa 6 bis 14 einstellt, oder Blutplasma direkt verwendet,

außer bei Verwendung von Malz ein lipolytischesamylytisches-proteolytisches Enzymgemisch zugibt, das aus etwa gleichen Anteilen jeweils eines aus Tier, Pflanze, Pilz und Bakterien gewonnen Enzyms besteht,

unter gleichmäßigem Rühren einige Zeit bei 30-60$^{o}$ C hält, dann zur Desaktivierung der Enzyme auf etwa 90 $^{o}$C erhitzt und heiß filtriert,

das Filtrat im Vakuum einengt und das Konzentrat einer Sprühtrocknung unterwirft,

das bei der Sprühtrocknung erhaltene Pulver in einem Alkohol/Wasser-Gemisch suspendiert, die Suspension einige Stunden stehen läßt, dann die überstehende Lösung vom Bodenkörper abfiltriert, das Filtrat konzentriert oder zur Trockne einengt und gegebenenfalls das so erhaltene Produkt in einem Alkohol/Wasser-Gemisch, vorzugsweise in einem 70/30 Alkohol/Wasser-Gemisch, gegen Wasser dialysiert.